# EUROPEAN PATENT SPECIFICATION

(11) **EP 3 697 413 B1**
(45) Date of publication and mention of the grant of the patent: **26.02.2025**
(21) Application number: 18868365.0
(22) Date of filing: 16.10.2018
(51) Int. Cl.: A61K 31/4188, A61K 31/685, A61N 5/10

(54) **COMBINATION THERAPY OF FRACTIONATED RADIATION AND SAPC-DOPS FOR THE TREATMENT OF TUMORS**
KOMBINATIONSTHERAPIE AUS FRAKTIONIERTER STRAHLUNG UND SAPC-DOPS ZUR BEHANDLUNG VON TUMOREN
THÉRAPIE COMBINATOIRE DE RAYONNEMENTS FRACTIONNÉS ET DE SAPC-DOPS POUR LE TRAITEMENT DE TUMEURS

(30) Priority: 16.10.2017 US 201762572729 P
(43) Date of publication of application: 26.08.2020
(73) Proprietor: University of Cincinnati, Cincinnati, OH 45221 (US)
(72) Inventor: QI, Xiaoyang, Cincinnati OH 45241 (US); DAVIS, Harold, W., Loveland OH 45140 (US)
(74) Representative: Moore, Michael Richard
(86) International application number: PCT/US2018/056006
(87) International publication number: WO 2019/079245

(56) References cited:
- WO-A1-2017/173288
- US-A1- 2015 290 300
- US-A1- 2016 367 670
- US-A1- 2017 202 965
- WOJTON JEFFREY ET AL.: "Systemic Delivery of SapC-DOPS Has Antiangiogenic and Antitumor Effects Against Glioblastoma", MOLECULAR THERAPY, vol. 21, no. 8, 1 August 2013 (2013-08-01), pages 1517 - 1525, XP055597426, ISSN: 1525-0016, DOI: 10.1038/mt.2013.114

## Description

### TECHNICAL FIELD

The present disclosure relates to the field of anti-cancer therapeutics and more particularly to methods for the treatment of tumors.

### BACKGROUND

Glioblastoma is the most common primary CNS malignant neoplasm in adults, and accounts for nearly 75% of the cases. Although there has been steady progress in their treatment due to improvements in neuro-imaging, microsurgery, and radiation, glioblastomas remain incurable. Glioblastomas cause death due to rapid, aggressive, and infiltrative growth in the brain. Glioblastomas are also relatively resistant to radiation and chemotherapy, and therefore posttreatment recurrence rates are high. In addition, the immune response to the neoplastic cells is mainly ineffective in completely eradicating residual neoplastic cells following resection and radiation therapy.

Normal living cells exhibit phosphatidylserine (PS) primarily within the intracellular leaflet of the plasma membrane. In contrast, viable cancer cells have high levels of PS on the external surface, and exhibit a broad range of surface PS, even within specific types of cancer. Agents that target surface PS have recently been developed to treat tumors and are expected to be more effective with higher surface PS levels.

Phospholipids are arranged asymmetrically in cell membranes with neutral phospholipids on the outer leaflet and anionic phospholipids (e.g. phosphatidylserine (PS) and phosphatidylethanolamine) located primarily on the inside of the membrane. An early event in apoptosis is the appearance of phosphatidylserine (PS) on the surface of the cell. This PS alerts phagocytic cells to engulf the cell and thus reduces the inflammatory response. Elevated external PS is also found in non-apoptotic primary and metastatic cancer cells, and in their associated tumor vasculature. Furthermore, viable cancer cells with high external PS are resistant to phagocyte-mediated removal. Macrophages recognize PS on the surface of apoptotic cells, but cancer cells appear to repel macrophages by displaying CD47, which inhibits phagocytosis. Recent studies have indicated that this surface PS may be exploited as a significant target for cancer therapy.

The loss of PS asymmetry in cancer cells may be due to reduced activity of ATP-dependent phospholipid translocases (flippases) and/or elevated activity of phospholipid scramblase, perhaps related to high levels of intracellular calcium (Ca²⁺ᵢ). While cancer cells generally have higher amounts of PS on the surface, the amount varies greatly among cultured cell lines, even within the same class of cancer. Cancer cells with high external PS have reduced flippase activity and high Ca²⁺ᵢ. compared to cancer cells with lower surface PS. The high surface PS cells also have elevated total cellular PS. Importantly, this increased PS is inducible and not associated with programmed cell death.

Radiotherapy is a common treatment for malignant tumors and has been shown to improve outcomes when used with chemotherapy. However, radiation can damage off target tissues and there is evidence that it can promote tumor growth by stimulating angiogenesis and cancer cell migration. Interestingly, radiation increases surface PS on tumor blood vessels and this has been successfully employed in mouse models of lung cancer and glioblastomas with the PS-targeting antibody, bavituximab.

US 2015/290300 relates to a composition comprising SapC-DOPS for use in a method of treating a malignant solid tumour in the brain of a patient. The composition includes SapC-DOPS administered in conjunction with rapamycin.

US 2017/202965 relates to methods for the treatment of cancer with a combination of radiation, cerium oxide nanoparticles and at least one chemotherapeutic agent.

An ongoing need exists for improved cancer therapies.

### SUMMARY

The invention and its most advantageous embodiments are defined in the appended set of claims.

As SapC-DOPS performs better with high surface PS cells, high surface PS cells selected by irradiation may decrease the effects of subsequent irradiation or even chemotherapy, but enhance susceptibility to SapC-DOPS treatment, thus introducing a potent new combination therapy.

The following summary of the disclosure is provided to facilitate an understanding of some of the innovative features unique to the present disclosure and is not intended to be a full description. A full appreciation of the various aspects of the invention can be gained by taking the entire specification, claims, drawings, and abstract as a whole.

In a first aspect, there is provided a composition comprising saposin C and dioleoylphosphatidylserine (SapC-DOPS) for use in a method of treating a malignant solid tumor in a patient in need thereof, the method comprising administering to the patient a combination of SapC-DOPS and fractionated radiation therapy, wherein the fractionated radiation therapy comprises a plurality of radiation fractions, each of said fractions delivering a radiation dose lower than a dose effective to inhibit tumor growth when administered alone, and further wherein one or more fractions of the fractionated radiation therapy are administered prior to the SapC-DOPS, and: (a) one or more additional fractions are administered after the SapC-DOPS; (b) one or more additional fractions are administered contemporaneously with the SapC-DOPS; and/or (c) SapC-DOPS and additional fractions are administered in overlapping or alternating sequences.

Described herein but not claimed is a method of treating a malignant solid tumor in a patient in need thereof, the method comprising administering to the patient a combination therapy comprising: fractionated radiation therapy and a therapeutically effective amount of saposin C and dioleoylphosphatidylserine (SapC-DOPS), wherein the fractionated radiation therapy comprises a plurality of radiation fractions, each of said radiation fractions delivering a radiation dose lower than a dose effective to inhibit tumor growth when administered alone.

Also described is a method of enhancing anti-tumor efficacy of SapC-DOPS in a subject suffering from a malignant solid tumor, the method comprising: administering to the subject a therapeutically effective amount of SapC-DOPS in combination with fractionated radiation therapy, wherein the fractionated radiation therapy comprises a plurality of radiation fractions administered over a treatment period, each of said fractions delivering a radiation dose lower than a dose effective to inhibit tumor growth when administered alone, wherein the fractionated radiation therapy enhances the anti-tumor efficacy of SapC-DOPS.

### BRIEF DESCRIPTION OF FIGURES

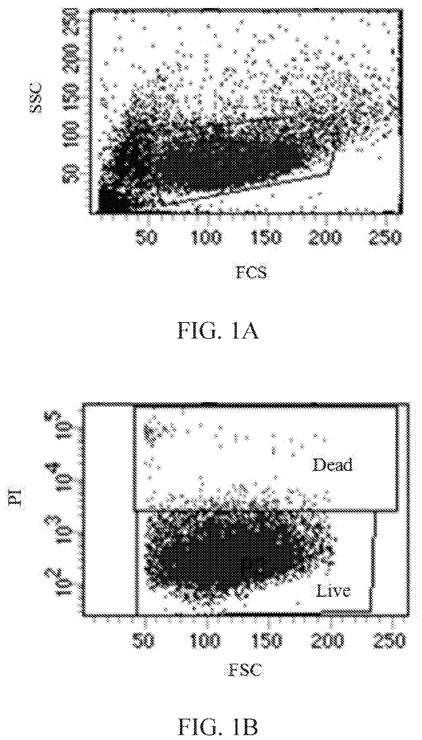

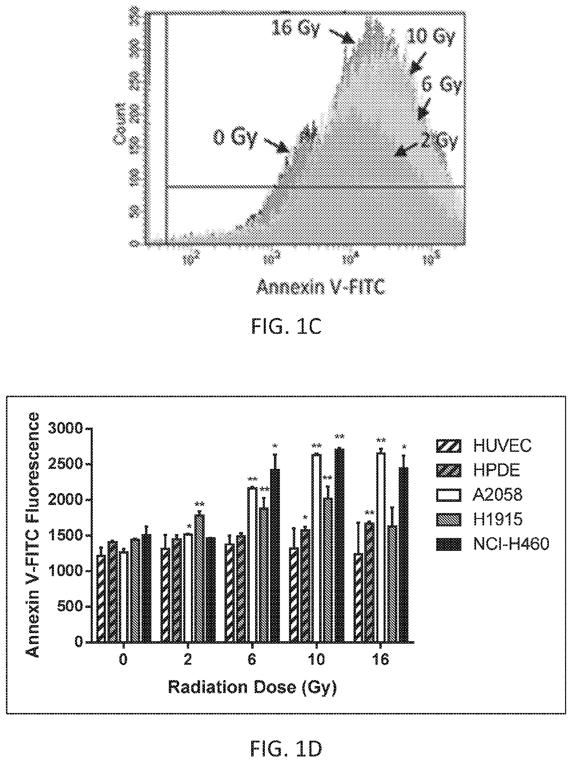

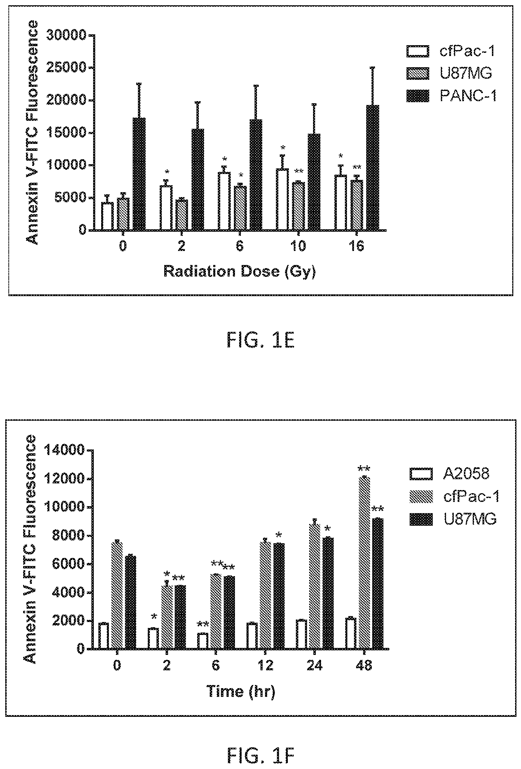

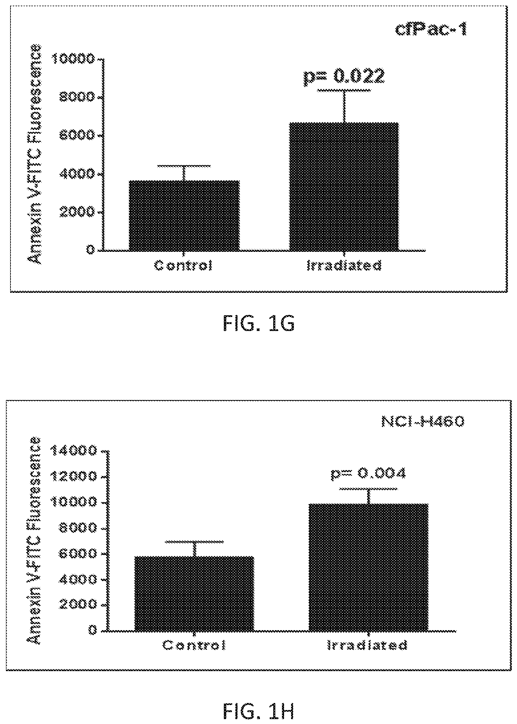

**FIG. 1A-C** )cfPac-1 cells, a human pancreatic cancer cell line, were irradiated at 0, 2, 6, 10 and 16 Gy. 24 hr later Annexin V-FITC staining was performed and the cells were processed by flow cytometry. **A)** Gating of cells. FSC: forward light scattering, an indication of cell volume; SSC: side light scattering, an indication of cellular granularity. **B)** Gating of live cells. PI (propidium iodide) stains only dead cells. **C)** Histogram of cells stained with Annexin V-FITC shows the effects of increasing doses of radiation on Annexin V staining (i.e. surface PS). **D-H)** Cells were irradiated at the indicated doses and 24 hr later Annexin V-FITC staining was determined on low surface PS cells (less than 2000 fluorescent units, **D)** or moderate or high surface PS cells (greater than 3000 fluorescent units, **E). F)** Cells were irradiated at 5 Gy and analyzed at the indicated times (A2058, cfPac-1 and U87MG). To examine whether tumor cell surface PS was also increased by radiation 2×10⁶ cfPac-1 **(G)** or NCI-H460 **(H)** cells were injected subcutaneously into nude mice. When the tumors were ~400 mm³ some were treated with 10 Gy of targeted irradiation as described in the Example section. The tumors were removed 48 hr. later and the cells were dispersed into single cells. The cells in all panels were then processed and gated as in FIG. 1. * p < 0.05, ** p < 0.01. cfPac-1 and PANC-1 are pancreatic cancer cell lines; A2058 is a melanoma cell line; NCI-H460 and H1915 are metastatic lung cancer cell lines; U87MG is a glioblastoma cell line, HPDE is a normal, immortalized pancreatic cell line and HUVEC are primary human umbilical vein endothelial cells.

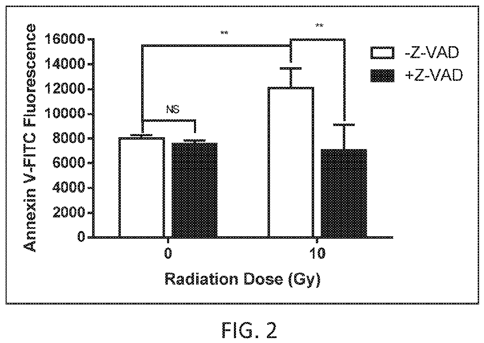

**FIG. 2** shows cfPac-1 cells that were irradiated at 10 Gy in the presence or absence of 10 µM Z-VAD-fink, Sigma (St. Louis, MO). 24 hr. later the cells were assessed for Annexin V binding as in FIG. 1. ** p < 0.01, NS = not significantly different from control.

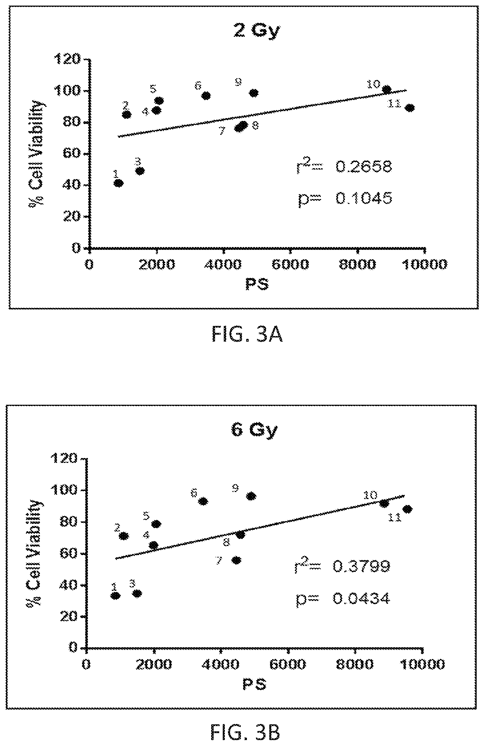

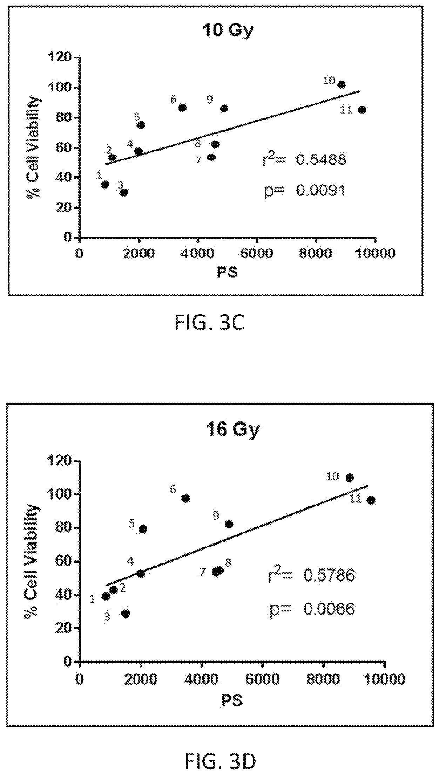

**FIG. 3** shows surface PS from untreated cells was determined with Annexin V-FITC staining and cell viability was ascertained with the MTT assay. Each dot represents the average of two measurements. r² and the p values were calculated with GraphPad Prism 6 software. A) shows results at 2 Gy; **B)** shows results at 6 Gy); **C)** shows results at 10 Gy; and **D)** shows results at 16 Gy.

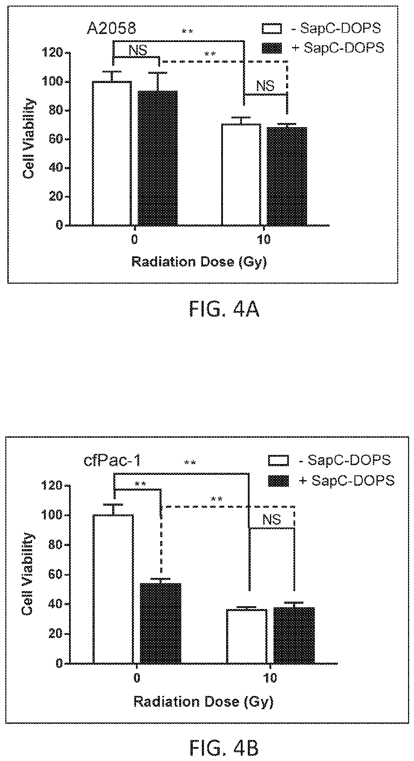

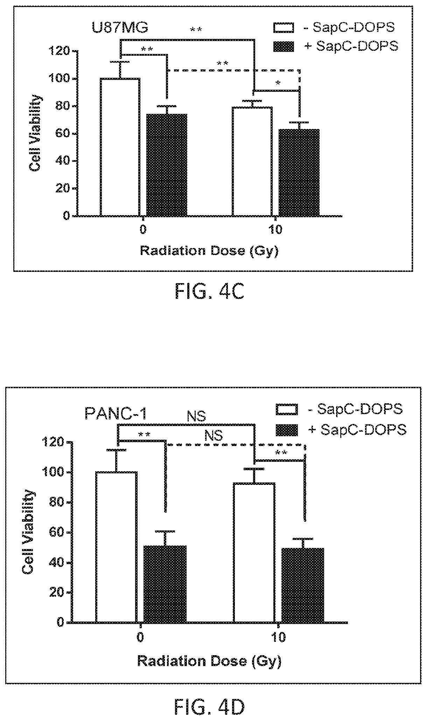

**FIG. 4** Cells were irradiated at 10 Gy and 24 hours SapC-DOPS was added. Cell death was analyzed 72 hr. later with the MTT assay. The SapC-DOPS concentration was 25 µM for A2058, cfPac-1 and PANC-1 but 40 µM for U87MG. Shown is the % of live cells compared to the control (no irradiation, no SapC-DOPS) for each cell line. * p < 0.05, ** p < 0.01, NS = not significantly different from control. **A**) shows results for A2058 cells; **B)** shows results for cfPac-1 cells; **C)** shows results for U87MG cells; and **D)** shows results for PANC-1 cells.

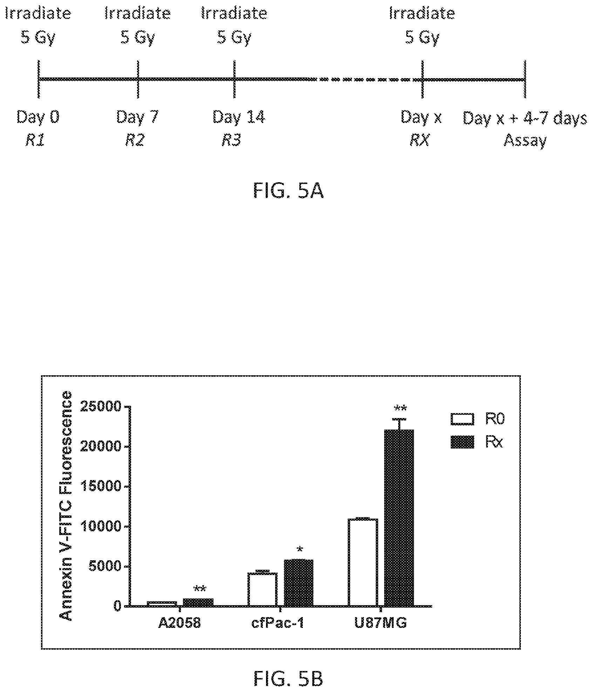

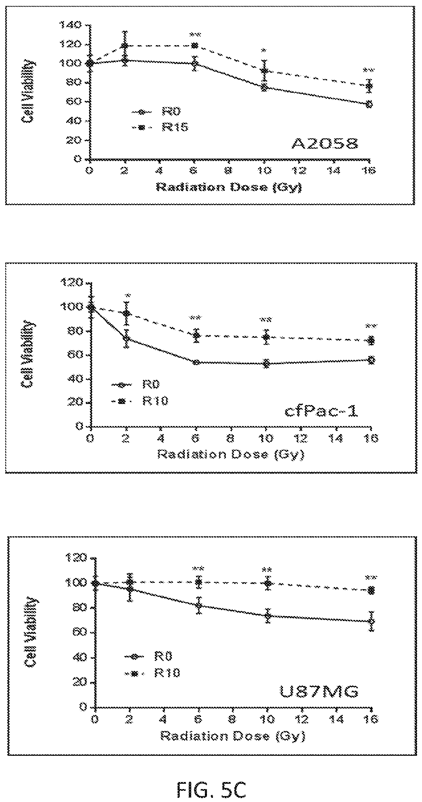

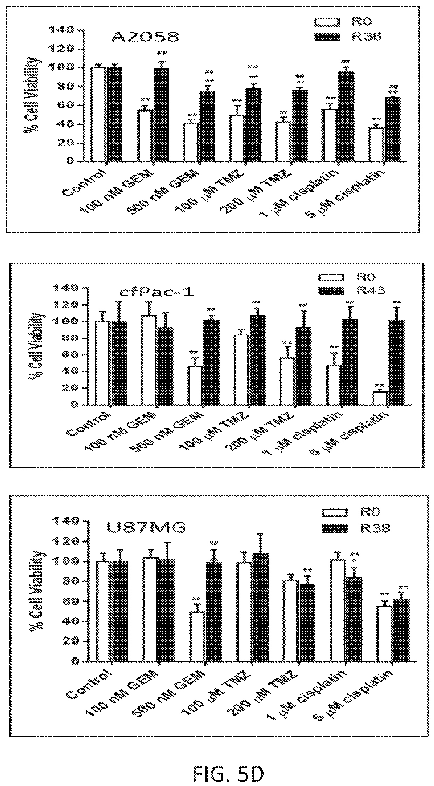

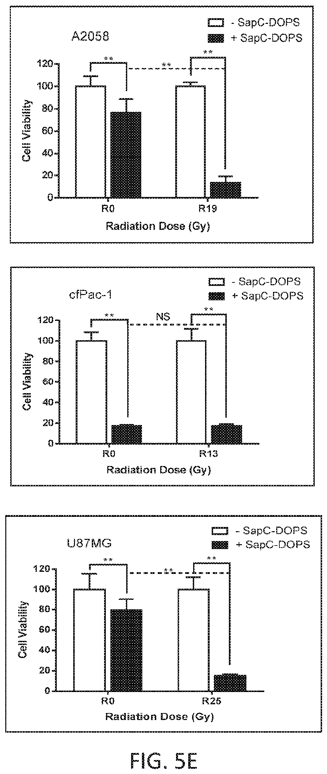

**FIG. 5** shows results of treatment of A2058, cfPac-1 and U87MG cell lines irradiated at 5 Gy every week for various numbers of weeks (Rx refers to the number of weeks the cells were irradiated). Non serially-irradiated cells are designated R0. Media was changed immediately after the irradiation and the cells were split every 3-4 days. Treatments were given 4-7 days following the last 5 Gy irradiation, when the surface PS is still elevated. **A)** Timeline of the treatment regime. **B)** Surface PS was determined by Annexin V-FITC staining on Day 4 following the last 5 Gy irradiation. Rx; x = 15 for A2058 and 10 for cfPac-1 and U87MG. * p < 0.05, ** p < 0.01 compared to R0. Shown are the mean and S.D. **C-E)** Cells were serially irradiated with 5 Gy. (top panel, A2058 cells; middle panel, cfPac-1 cells; bottom panel, U87MG cells) **C)** 7 days later they were exposed to the indicated doses of radiation and cell viability was determined by MTT assay was performed 72 hr. later. Shown is the % of live cells with 100% as the normalized control for each cell line. * p < 0.05, ** p < 0.01, compared to R0. **D)** 7 days after the last 5 Gy irradiation, cells were treated with different doses of gemcitabine (GEM) temozolomide (TMZ) or cisplatin and cell viability was determined by MTT assay was performed 72 hr. later. ^{#}p < 0.05, ^{##} p < 0.01, compared to R0 at the same dose of drug. **E)** 7 days following the last 5 Gy irradiation, cells were treated with the indicated doses of SapC-DOPS (1 SapC:7 DOPS, Mol:Mol) and cell viability was determined by MTT assay was performed 72 hr. later. SapC-DOPS concentration was 25 µM for A2058, cfPac-1 and PANC-1 but 40 µM for U87MG. Shown are the % of live cells with 100% as the normalized control for each cell line (as well as for the R0 and Rx). * p < 0.05, ** p < 0.01, NS = not significantly different.

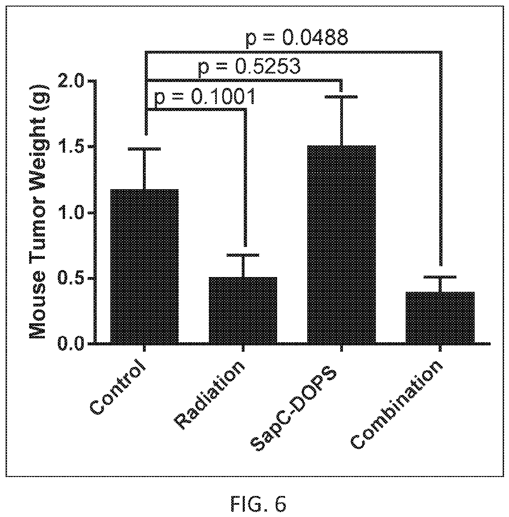

**FIG. 6** shows results of combination therapy of radiation and SapC-DOPS on mice injected with 1×10⁶ NCI-H460 cells. When tumors reached ~100 mm³, the mice were irradiated at 10 Gy on days 9 and 16 following injection of the cells. SapC-DOPS (4 mg/kg) was injected via the tail vein on days 11, 14, 15, 16, 17 and 18. The mice were euthanized on day 22 and the tumors were removed, rinsed with saline and weighed. p values from t-tests are shown.

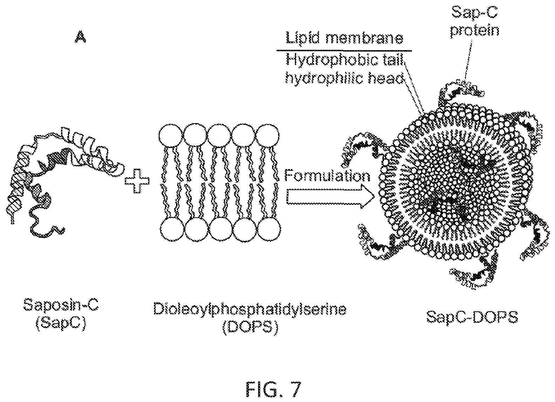

**Fig. 7** depicts the structure of SapC-DOPS.

### SEQUENCE LISTING

Applicant hereby refers to a CRF sequence listing submitted herewith having a file name 10738-681_Sequence_Listing.txt created on October 15, 2018.

The nucleic and amino acid sequences listed in the accompanying sequence listing are shown using standard letter abbreviations for nucleotide bases as defined in 37 C.F.R. 1.822. Only one strand of each nucleic acid sequence is shown, but the complementary strand is understood as included by any reference to the displayed strand. In the accompanying sequence listing:
SEQ ID NO: 1 represents a peptide sequence of saposin C.

### DETAILED DESCRIPTION

The following description of particular embodiment(s) is merely exemplary in nature and is in no way intended to limit the scope of the invention, its application, or uses, which may, of course, vary. The invention is described with relation to the non-limiting definitions and terminology included herein. These definitions and terminology are not designed to function as a limitation on the scope or practice of the invention but are presented for illustrative and descriptive purposes only.

The terminology used herein is for the purpose of describing particular embodiments only and is not intended to be limiting. As used herein, the singular forms "a," "an," and "the" are intended to include the plural forms, including "at least one," unless the content clearly indicates otherwise. "Or" means "and/or." As used herein, the term "and/or" includes any and all combinations of one or more of the associated listed items. It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof. The term "or a combination thereof" means a combination including at least one of the foregoing elements.

Unless otherwise indicated, all numbers expressing quantities of ingredients, properties such as reaction conditions, and so forth used in the specification and claims are to be understood as being modified in all instances by the term "about." Accordingly, unless indicated to the contrary, the numerical parameters set forth in this specification and claims are approximations that can vary depending upon the desired properties sought to be obtained by the presently-disclosed subject matter.

As used herein, the term "about," when referring to a value or to an amount of mass, weight, time, volume, pH, size, concentration or percentage is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed method.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Unless otherwise defined, all terms (including technical and scientific terms) used herein have the same meaning as commonly understood by one of ordinary skill in the art to which this disclosure belongs. It will be further understood that terms such as those defined in commonly used dictionaries should be interpreted as having a meaning that is consistent with their meaning in the context of the relevant art and the present disclosure, and will not be interpreted in an idealized or overly formal sense unless expressly so defined herein.

As used herein, "malignant solid tumor" refers to an abnormal mass of cancerous cells that typically does not contain cysts or liquid areas. In embodiments, the solid tumor is a brain, pancreatic, lung, appendiceal , or neuroblastoma tumor. In some embodiments, the tumor is a brain tumor, such as a glioblastoma. As used herein, "glioblastoma multiforme," "GBM," and "glioblastoma" refer to grade IV primary glioma brain cancer.

As used herein a "subject" or "patient" refers to a mammal. Optionally, a subject or patient is a human or non-human primate. Optionally, a subject or patient is a dog, cat, horse, sheep, cow, rabbit, pig, or mouse.

As used herein, the term "sequentially" refers to a treatment protocol in which administration of a first therapeutic agent or therapy is followed by administration of a second therapeutic agent or therapy. In embodiments, the therapy comprises radiation therapy, including radiation fractions.

As used herein, the term "contemporaneously" refers to administration of a first therapeutic agent or therapy and administration of a second therapeutic agent or therapy, wherein the first and second therapeutic agents or therapies are separate and are administered at substantially the same time. In embodiments, the therapy comprises radiation therapy, including radiation fractions.

The term "pharmaceutically-acceptable excipient," as used herein, means any physiologically inert, pharmacologically inactive material known to one skilled in the art, which is compatible with the physical and chemical characteristics of the particular active agent selected for use. Pharmaceutically acceptable excipients include, but are not limited to, polymers, resins, plasticizers, fillers, lubricants, diluents, binders, disintegrants, solvents, co-solvents, buffer systems, surfactants, preservatives, sweetening agents, flavoring agents, pharmaceutical grade dyes or pigments, and viscosity agents.

A "therapeutically effective amount" is defined herein in relation to the treatment of cancers as an amount that will decrease, reduce, inhibit, or otherwise abrogate the growth of a cancer cell or tumor. In some embodiments, the therapeutic agent(s) can be delivered regionally to a particular affected region or regions of the subject's body. In some embodiments, wherein such treatment is considered more suitable, the therapeutic agent(s) can be administered systemically. For example, the compound can be administered orally or parenterally. In a specific embodiment, a therapeutic agent is delivered intravenously.

The terms "treat," "treatment," and "treating," as used herein, refer to a method of alleviating or abrogating a disease, disorder, and/or symptoms thereof in a subject.

SapC-DOPS refers to a stable nanovesicle composed of saposin C (SapC), a lysosomal protein that catabolizes glycosphingolipids, and the phospholipid dioleoylphosphatidylserine (DOPS) (**Fig. 7**). SapC-DOPS is further described in U.S. Patent No. 8,937,156, issued January 20, 2015. In one embodiment, SapC has a protein sequence consisting of SDVYCEVCEFLVKEVTKLIDNNKTEKEILDAFDKMCSKLPKSLSEECQEVVDTYGSSILSI LLEEVSPELVCSMLHLCSG (SEQ ID NO: 1). In some embodiments, SapC comprises a protein sequence having at least 75%, at least 80%, at least 85%, at least 90%, at least 95%, at least 98%, or at least 99% sequence identity to SEQ ID NO: 1.

The term "fractionated radiation" refers to a radiotherapy, wherein a total dose of radiation is divided across a number of smaller doses, called radiation fractions, which are administered over a treatment period. In some embodiments, each fraction delivers a radiation dose lower than a dose effective to inhibit tumor growth when administered alone. In embodiments, a total dose of radiation ranges from about 20 to about 80 Gray (Gy). In embodiments, fractionated radiation therapy comprises from about 20 to about 40 fractions, from about 25 to about 35, or about 30 fractions. In embodiments, a radiation fraction comprises from about 1 to about 10 Gy, from about 1 to about 15 Gy, from about 1 to about 12 Gy, from about 1 to about 10 Gy, from about 1 to about 8 Gray, from about 1 to about 5 Gy, or from about 1 to about 2 Gy.

"Treatment period" refers to a length of time corresponding to a therapeutic treatment regimen. In some embodiments, a combination of SapC-DOPS and fractionated radiation is administered over a period of time ranging from days to weeks or months. In some embodiments, a treatment period comprises from about 20 to about 40 consecutive days.

The present disclosure shows that co-treatment of U87-MG glioblastoma cells with a fractionated radiation therapy and SapC-DOPS demonstrates synergistic anti-proliferative effects when compared to either treatment alone. The present disclosure indicates that surface PS elevation that increases as a result of radiation is decreased with the treatment of SapC-DOPS, and that combined therapy increases the rate of cell death of the malignant solid tumor in a patient.

### Methods of Treatment

Described herein is a method of treating a malignant solid tumor in a patient in need thereof, the method comprising administering to the patient a combination therapy comprising: fractionated radiation therapy, wherein the fractionated radiation therapy comprises a plurality of radiation fractions, each of said fractions delivering a radiation dose lower than a dose effective to inhibit tumor growth when administered alone; and a therapeutically effective amount of SapC and dioleoylphosphatidylserine (SapC-DOPS).

Also described herein is a method of enhancing anti-tumor efficacy of SapC-DOPS in a subject suffering from a malignant solid tumor, the method comprising: administering to the subject a therapeutically effective amount of SapC-DOPS in combination with fractionated radiation therapy, wherein the fractionated radiation therapy comprises a plurality of radiation fractions administered over a treatment period, each of said fractions delivering a radiation dose lower than a dose effective to inhibit tumor growth when administered alone, wherein the fractionated radiation therapy enhances the anti-tumor efficacy of SapC-DOPS.

In some embodiments, the fractionated radiation therapy comprises a plurality of radiation fractions administered over a treatment period. In one or more embodiments, the plurality of radiation fractions includes about 20 to about 40 fractions. In one or more embodiments, the fractionated radiation therapy comprises from about 25 to about 35 fractions. In one embodiment, the fractionated radiation therapy includes about 30 fractions. In one or more embodiments, each fraction may include a radiation dose of from about 1 gray (Gy) to about 2 Gy.

In some embodiments, the fractionated radiation therapy comprises a total radiation dose of from about 20 Gy to about 80 Gy. Since fractionated radiation therapy includes multiple fractions of radiation, the fractionated radiation is administered over a treatment period. In some embodiments, the treatment period comprises from about 20 to about 40 consecutive days.

In embodiments, the therapeutically effective amount of SapC-DOPS that is administered in a plurality of doses over the treatment period. In specific embodiments, SapC-DOPS is administered in 10 to 20 doses over the treatment period. In other specific embodiments, SapC-DOPS is administered in 10 to 15 doses over the treatment period.

Each individual dose of the plurality of doses of SapC-DOPS may comprise an amount of from 0.1 mg/kg to 5.0 mg/kg SapC. In some embodiments, the individual dose comprises an amount of from 0.2 mg/kg to 3.0 mg/kg SapC. In other embodiments, the individual dose comprises an amount of from 0.3 to 1.2 mg/kg SapC.

In accordance with the invention, one or more fractions are administered before administering the SapC-DOPS composition. In accordance with the invention, one or more fractions are administered after administering the SapC-DOPS composition in one or more doses. In accordance with the invention, one or more fractions are administered contemporaneously with administration of the SapC-DOPS composition.

In accordance with the invention, administering the radiation therapy and administering the SapC-DOPS is accomplished in overlapping or alternating sequences. For example, a fraction of radiation may be administered to the patient on Day 1, then on Day 2, a fraction of radiation is administered to the patient together with a dose of SapC-DOPS. Additionally, SapC-DOPS may be administered to the patient on Day 1, and on Day 2 and Day 3 the patient is administered a radiation fraction.

In some embodiments, the therapeutically effective amount of SapC-DOPS is administered in 10 to 15 doses over a twenty-eight day cycle. In an exemplary schedule, a dose of SapC-DOPS is administered five times during the first week; three times during weeks two and three; and once during week four.

In some embodiments, the SapC-DOPS is administered parenterally. In a specific embodiment, SapC-DOPS is administered to the patient via an intravenous route.

In various embodiments, malignant solid tumor is a brain, pancreatic, lung, or neuroblastoma tumor. In one or more embodiments, the brain tumor is glioblastoma. In certain embodiments, the method ameliorates at least one symptom of glioblastoma in a mammal selected from the group consisting of mouse, rat, dog, cat, monkey, and human.

In certain embodiments of the composition for use in a method, the method further comprises administering an additional anticancer agent to the patient or tumor, wherein the anticancer agent is selected from the group consisting of: chemotherapeutic agents; cytotoxins; antimetabolites; alkylating agents; protein kinase inhibitors; anthracyclines; antibiotics; antimitotic agents; corticosteroids; radiopharmaceuticals; cytokines; enzymes; interferons; krestin; lentinan; sizofiran; picibanil; ubenimex; acitretin; fenretinide; thalidomide; zoledronic acid; angiostatin; aplidine; cilengtide; combretastatin A-4; endostatin; halofuginone; rebimastat; removab; Revlimid; squalamine; ukrain; Vitaxin; cisplatin; carboplatin; nedaplatin; oxaliplatin; camptothecin derivatives; compounds or chelates that include radionuclides; filgrastim; lentinan; sizofilan; TheraCys; ubenimex; WF-10; aldesleukin; alemtuzumab; BAM-002; dacarbazine; daclizumab; denileukin; gemtuzumab ozoganmicin; ibritumomab; imiquimod; lenograstim; lentinan; Corixa; molgramostim; OncoVAX-CL; sargramostim; tasonermin; tecleukin; thymalasin; tositumomab; Virulizin; Z-100; epratuzumab; mitumomab; oregovomab; pemtumomab; Provenge; alitretinoin; ampligen; atrasentan bexarotene; bortczomib; Bosentan; calcitriol; exisulind; finasteride fotemustine; ibandronic acid; miltefosine; mitoxantrone; 1-asparaginase; procarbazine; dacarbazine; hydroxycarbamide; pegaspargase; pentostatin; tazarotne; Telcyta; Velcade; tretinoinor; macitentan; carmustine; (R)-7-Acetyl-5-(4-aminophenyl)-8,9-dihydro-8-methyl-7H-I,3-dioxolo[4,5-H][2,3]benzodiazepine; paclitaxel; temazolomide; gemcitabine; and combinations thereof.

The skilled artisan will appreciate that the dosing schedules and amounts set forth herein are exemplary and may be varied by the attending physician in accordance with the age and physical condition of the subject to be treated, the severity of the disease, the duration of the treatment, the nature of concurrent therapy, the particular combination of therapeutic agents being employed, the particular pharmaceutically-acceptable excipients utilized, and like factors within the knowledge and expertise of the attending physician.

### EXAMPLES

The effects of single and serial dose irradiation on the surface PS of a number of cancer cells were examined. In the clinic, fractionated radiation therapy is often used to protect the patients from a single high dose radiation exposure. Therefore, the cells were serially irradiated at 5 Gy once per week for several weeks to investigate whether this would alter surface PS or modify the effects obtained with a single dose of radiation.

### Example 1: Materials and Procedures

### Cell lines

Human cancer cell lines, A2058, AsPC-1, SKNSH, MiaPaCa-2, cfPac-1, NCI-H460, H1915, H1299, U87MG and U373 and the normal pancreatic cell line (HPDE) were obtained from ATCC (Manassas, VA). MDA-MB-231-Luc were obtained from Caliper Life Sciences (Mountain View, CA). HUVEC were from Lonza (Basel, Switzerland). HPDE, A2058, AsPC-1, MiaPaCa-2, cfPac-1, U87MG, Gli36 and U373 were cultured in DMEM (Fisher Scientific, Pittsburgh, PA). H460, H1299 and H1915 were cultured in RPMI 1640 (Fisher Scientific). MDA-MB-231- Luc and SKNSH were cultured in AMEM (Invitrogen, Carlsbad, CA). The above cell lines except HUVEC, were cultured in their respective media supplemented with 10% FBS and 1% Penicillin/Streptomycin. HUVEC were grown in EGM-2 media (Lonza).

### Flow cytometric analyses of annexin V binding

Unless otherwise stated, cells at 60-80% confluency were irradiated and then, after the appropriate time, were trypsinized, resuspended in complete medium, spun down, and washed once with PBS then resuspended in annexin V staining buffer. Cells (1 × 10⁵) were incubated at room temperature in the dark for 15 minutes in a final volume of 100 µl containing 10 µl Annexin V-FITC (Invitrogen, Carlsbad, CA) and 2 µg/ml propidium iodide (PI; BD-Pharmingen, Eugene, OR). Annexin V-FITC binding was measured by flow cytometry after adding 400 µl of annexin V binding buffer, using a BD Fortessa. Data was analyzed with BD FACS Diva. For quantifying the annexin V-FITC signal from living cells, PI positive dead cells were gated out and the annexin V-FITC signal was obtained from PI negative forward scattered cells.

### Cell viability assay

Cells were seeded in flat bottom 96 well plates (Falcon, Becton Dickson Labware, Franklin Lakes, NJ) at 2000-12000 cells/per well (depending on the growth rate of the cell line). The number of cells seeded was adjusted to give an optical density of 0.6 to 1.2 (linear range of the assay) at the time of testing. The next day the cells were treated and 72 hr. later were analyzed for cell viability with the Cell Proliferation Kit I (MTT; Roche Diagnostics, Mannheim, Germany).

### Flippase and Scramblase assays

Flippase was measured as previously described (Vallabhapurapu SD, Blanco VM, Sulaiman MK, Vallabhapurapu SL, Chu Z, Franco RS, Qi X: Variation in human cancer cell external phosphatidylserine is regulated by flippase activity and intracellular calcium. Oncotarget 2015, 6(33):34375-34388). Briefly, cells were resuspended in 3 ml of flippase assay buffer and NBD-PS (Avanti Polar Lipids) was added to a final concentration of 3 µM. Cells with NBD-PS were divided into aliquots and incubated for 0, 1, 5, 15, 30 or 45 minutes. After each incubation time, half of the cell suspension was separated for non-extracted sample and kept on ice. The remaining half was spun down to remove non inserted NBD-PS and subjected to BSA extraction of NBD-PS from the outer leaflet by adding 3% fatty acid free BSA (MP Biomedicals) in flippase assay buffer. After incubation on ice for 20 minutes, freshly prepared sodium dithionite (Sigma) was added to a final concentration of 10 mM and incubated a further 10 minutes, to reduce the NBD lipids in the outer cell surface. The cells were spun down and resuspended in flippase assay buffer containing 0.25% BSA and 2 µg/ ml PI. NBD-PS signal from unextracted and extracted samples was measured by flow cytometry from living cells after exclusion of PI-positive dead cells, using a BD Fortessa. Non-extractable NBD-PS in the BSA and sodium dithionite treated sample was presented as the percentage of total amount in the control unextracted sample. Scramblase was measured in the same way but with NBD-PC (Avanti Polar Lipids).

### Measurement of intracellular calcium

Intracellular calcium was measured by using the calcium binding dye Fluo-3 AM (Invitrogen). Cells (1 × 10⁵) were loaded with 5 µM final concentration of Fluo-3 AM and incubated for 30 min at 37 °C. Cells were washed twice with DMEM, and resuspended in DMEM and incubated for a further 30 minutes at 37 °C. The cells were washed twice with PBS +2% FBS and resuspended in 400 µl of this. Two µg/ml PI was added and the Fluo-3 AM signal was measured by flow cytometry after exclusion of PI positive dead cells, using a BD Fortessa cytometer. Data was analyzed using BD FACS Diva software.

### Thin layer chromatography (TLC) and quantification of total phosphatidylserine

Total cellular lipids were extracted by chloroform/methanol extraction. TLC was performed as previously described (Vallabhapurapu SD, Blanco VM, Sulaiman MK, Vallabhapurapu SL, Chu Z, Franco RS, Qi X: Variation in human cancer cell external phosphatidylserine is regulated by flippase activity and intracellular calcium. Oncotarget 2015, 6(33):34375-34388). Lipids were loaded onto a TLC plate based on protein quantification. Brain PS (Avanti Polar Lipids) and sphingomyelin (Matreya LLC) were run as molecular standards. Bands corresponding to PS were scraped and subjected to phosphorus extraction by acidic digestion. The liberated phosphorus was estimated by allowing a complex formation with ammonium molybdate (Sigma) and malachite green (Sigma) and by measuring the absorption at 660 nm (Zhou X, Arthur G: Improved procedures for the determination of lipid phosphorus by malachite green. J Lipid Res 1992, 33(8): 1233-1236). Phosphorus was quantified using a standard curve obtained from phosphorus liberated from known concentrations of brain PS run on TLC plate. Cellular PS was expressed as the ratio of phosphorus obtained from PS and phosphorus obtained from total phospholipids. Additionally, PS was estimated by acquiring TLC band intensities of PS and sphingomyelin, using Image Studio Lite software; variations in PS are shown as a ratio of PS to sphingomyelin bands.

### Tumor growth in mice

All animal studies were approved by the Institutional Animal Care and Use Committee of the University of Cincinnati (Protocol Number: 11-05-05-02). Female nude/nude athymic mice (Taconic Farms, Germantown, NY) weighting approximately 20-25 g were injected with cancer cells (1-2 × 10⁶ cells in 0.2 ml PBS) subcutaneously in the right shoulder or flank. When the tumors were approximately 400 mm³, they were irradiated with targeted radiation as below. After 48 hr. the tumors were removed, dissected, and dissociated from a 30 mg portion with a kit from 101Bio (Palo Alto, CA). The harvested cells were stained with Annexin V-FITC and PI and subjected to flow cytometry. In other experiments, mice were treated with SapC-DOPS (prepared as in Chu Z, Abu-Baker S, Palascak MB, Ahmad SA, Franco RS, Qi X: Targeting and cytotoxicity of SapC-DOPS nanovesicles in pancreatic cancer. PLoS One 2013, 8(10):e75507) following the irradiation and the tumor sizes were measured for up to 15 days.

To examine radiation and SapC-DOPS on tumor growth, mice were injected with 1×10⁶ NCI-H460 cells. When the tumors reached 100 mm³ the mice were treated with 10 Gy irradiation and/or 4 mg/kg SapC-DOPS. At the end of the experiment the tumors were removed and weighed.

### Irradiation

Cells were grown to 60-80% confluency in 60 mm dishes then exposed to (¹³⁷Cs) irradiation with a GammaCell 40 Exactor (Nordion International) at doses of 0, 2, 6, 10 or 16 Gy. The media was changed and the cells were processed for surface PS determination at the indicated times. Mice were irradiated at 10 Gy with an Xstrahl D3100 superficial systems (at 100 kV) with 2 cm cone and projected 1 cm deep.

### Statistical Analyses

Differences between treatment groups were determined by t-test, Pearson correlations were obtained with GraphPad Prism and data are presented as mean ± standard deviation

### Comparative Example 2: A single dose of irradiation increased the surface PS of cancer cells in vitro and in vivo

A pancreatic cancer cell line with moderate surface PS, cfPac-1, exhibited a radiation dose dependent increase in PS 24 hour after irradiation, as shown in the graph of **FIG. 1C****.** The increase in surface PS was indicated on PI negative live cells as little cell death was detected during the first 48 to 72 hours after the irradiation. This effect occurred when the radiation dose was 16 Gy. Although cell lines varied in their response, radiation increased overall surface PS populations in all of the cancer cells that were tested, with the exception of PANC-1, which has a very high initial level of surface PS (**FIGS. 1D** **and** **1E**). In contrast, HUVEC (human umbilical vein endothelial cells) and HPDE cells (a normal pancreatic epithelial cell line) had little or no increase in surface PS (**FIG. 1D**). For all cancer and normal cell lines the percentage of dead cells was less than 10% and did not change significantly in the first 48 hr following any of the radiation doses. The time dependency of PS change after irradiation was examined in several cancer cell lines, and an initial decrease was found before the increase (**FIG. 1F**).

An increase in cell surface PS was also detected after irradiation of subcutaneous tumors formed after injection of cfPac-1 (**FIG. 1G**) or NCI-H460 (**FIG. 1H**). Although there were variable numbers of dead cells associated with the tumors, this did not change appreciably with irradiation. For cfPac-1 the percentage of dead cells was 1.1 ± 0.6 and 2.7 ± 0.8 for control and irradiated cells, respectively; for NCI-H460 it was 72.0 ± 15.0 and 65.9 ± 2.2. All of the PS data shown are on live (propidium iodide negative) cells.

### Comparative Example 3: The increase in surface PS after a single irradiation is dependent on caspase activity.

The pan-caspase inhibitor, Z-VAD fmk, completely eliminated the radiation-induced surface PS elevation (**FIG. 2**). On the other hand, as shown in **Table 1,** the activities of flippase and scramblase are unchanged in cfPac-1 cells during the period when the cells are still responding to the 10 Gy irradiation by increasing surface PS. While there is a slight, insignificant decrease in scramblase activity, an increase in this activity would be expected if scramblases were involved in the radiation-induced increase in surface PS. Total PS and intracellular calcium were also unchanged (see **Table 1**).

**Table 1. The increase in surface PS caused by irradiation is unclear but does not appear to be due changes in intracellular calcium translocase activity or total PS.**

| | Units | Control | Irradiated (10 Gy) | |
|---|---|---|---|---|
| Flippase | Initial rate | 2.03 ± 0.02 | 2.03 ± 0.07 | NS |
| Scramblase | Initial rate | 0.75 ± 0.19 | 0.33 ± 0.4 | NS |
| Total PS | PS/PI ratio | 7.50 ± 2.57 | 5.28 ± 1.95 | NS |
| Calcium | Arbitrary fluorescence | 8717.3 ± 421.8 | 7245.5 ± 777.1 | NS |

The activities of the PS translocases, flippase and scramblase, the total amount of PS and intracellular calcium were determined in cfPac-1 cells 24 hr. after 10 Gy irradiation

### Comparative Example 4: Cancer cell surface PS is correlated with sensitivity to a single dose of irradiation

As presented in **FIG. 1C, FIG. 1D****,** **FIG. 1E,** and **FIG. 1F****,** there was a positive correlation between surface PS on cancer cells and their resistance to a single dose of irradiation. In addition, the data show that the higher the dose of radiation, the stronger the correlation. Since irradiation selects for cells with higher surface PS, irradiation may make tumors less sensitive to future irradiation. Interestingly, these results suggest that the amount of surface PS on clinical specimens may be used as a marker to indicate whether the tumor will be sensitive to irradiation. Further, the increase in surface PS may make the tumors more susceptible to the PS-targeting drug SapC-DOPS.

### Comparative Example 5: A single dose of irradiation has modest or no effect on SapC-DOPS-induced cell death.

Contrary to expectations, a single dose of 10 Gy, although it increased the proportion of cells with higher surface PS (see **FIGS. 1A - 1H**), did not enhance the cell killing ability of marginally effective doses of SapC-DOPS in either A2058 or cfPac-1 cells, and only showed modest augmentation in U87MG cells **(****FIGS. 4A** - **4D**). This may be due to the increased surface PS that occurs at the early stages of apoptosis. While not desiring to be bound by theory, it is understood that because these cells are already dying, there would be no additional cell death with SapC-DOPS. There was also no improvement of SapC-DOPS activity in PANC-1 cells, although PANC-1 cells already had a high surface PS, which was unaffected by radiation.

### Example 6: Serial irradiation of cancer cell lines makes them less sensitive to subsequent radiation and to chemotherapeutic drugs but more sensitive to SapC-DOPS

Since fractionated radiation therapy is a safer treatment regimen used routinely in the clinic, serial irradiation of cells were performed, weekly with 5 Gy for multiple weeks. During this time the cells were split every 3-4 days. The cells were analyzed 4-7 days following the final irradiation (see **FIG. 5A**). As shown in **FIG. 5B****,** the serially irradiated cells had significantly higher surface PS than non-irradiated cells. These cells became more resistant to a single subsequent dose of radiation **(****FIGS. 5C - 5E****)** or to chemotherapeutic drugs, i.e., gemcitabine, temozolomide and cisplatin (**FIGS. 5D**). However, serially irradiated A2058 and U87MG became more susceptible to SapC-DOPS treatment (**FIG. 5E**).

### Example 7: Combination treatment with radiation and SapC-DOPS reduces tumor growth better than either therapeutic alone

NCI-H460 cells injected into mice quickly form tumors. A radiation dose of 10 Gy and a dose of 4mg/kg of SapC-DOPS were selected did not significantly attenuate tumor growth when administered separately. However, when each treatment was administered in combination, there was a significant reduction in tumor size (**FIG. 6**).

## Claims

1. A composition comprising saposin C and dioleoylphosphatidylserine, SapC-DOPS, for use in a method of treating a malignant solid tumor in a patient in need thereof, the method comprising administering to the patient a combination of SapC-DOPS and fractionated radiation therapy, wherein the fractionated radiation therapy comprises a plurality of radiation fractions, each of said fractions delivering a radiation dose lower than a dose effective to inhibit tumor growth when administered alone, and further wherein one or more fractions of the fractionated radiation therapy are administered prior to the SapC-DOPS, and:
(a) one or more additional fractions are administered after the SapC-DOPS;
(b) one or more additional fractions are administered contemporaneously with the SapC-DOPS; and/or
(c) SapC-DOPS and additional fractions are administered in overlapping or alternating sequences.

2. The composition for use according to claim 1, wherein the fractionated radiation therapy comprises from about 20 to about 40 fractions.

3. The composition for use according to claim 1, wherein the SapC-DOPS is administered in a plurality of doses over a treatment period.

4. The composition for use according to claim 3, wherein the treatment period comprises from about 20 to about 40 consecutive days.

5. The composition for use according to claim 3, wherein the plurality of doses of SapC-DOPS comprises from about 10 to about 20 doses over the treatment period.

6. The composition for use according to claim 3, wherein a dose of SapC-DOPS comprises from about 0.3 mg/kg to about 1.2 mg/kg.

7. The composition for use according to claim 1, wherein the fractionated radiation therapy comprises a total radiation dose of from about 20 Gy to about 80 Gy.

8. The composition for use according to claim 1, wherein each fraction comprises a radiation dose of from about 1 Gy to about 2 Gy.

9. The composition for use according to claim 1, wherein the malignant solid tumor is a brain, pancreatic, lung, appendiceal, or neuroblastoma tumor.

10. The composition for use according to claim 1, wherein the SapC-DOPS is administered via an intravenous route.

11. The composition for use according to claim 1, wherein the SapC-DOPS is administered in 10 to 15 doses over a twenty-eight day treatment period.

12. The composition for use according to claim 1, wherein the method further comprises administration of an additional anticancer agent.

## Patentansprüche

1. Zusammensetzung, umfassend Saposin C und Dioleoylphosphatidylserin, SapC-DOPS, zur Verwendung in einem Verfahren zum Behandeln eines bösartigen soliden Tumors bei einem Patienten, der dies benötigt, das Verfahren umfassend ein Verabreichen einer Kombination aus SapC-DOPS und fraktionierter Strahlentherapie an den Patienten, wobei die fraktionierte Strahlentherapie eine Vielzahl von Strahlungsfraktionen umfasst, jede dieser Fraktionen eine Strahlungsdosis abgibt, die niedriger ist als eine Dosis, die bei alleiniger Verabreichung wirksam ein Tumorwachstum hemmt, und wobei ferner eine oder mehrere Fraktionen der fraktionierten Strahlentherapie vor dem SapC-DOPS verabreicht werden, und:
(a) eine oder mehrere zusätzliche Fraktionen nach dem SapC-DOPS verabreicht werden;
(b) eine oder mehrere zusätzliche Fraktionen gleichzeitig mit SapC-DOPS verabreicht werden; und/oder
(c) SapC-DOPS und zusätzliche Fraktionen in überlappenden oder abwechselnden Sequenzen verabreicht werden.

2. Zusammensetzung zur Verwendung nach Anspruch 1, wobei die fraktionierte Strahlentherapie von etwa 20 bis etwa 40 Fraktionen umfasst.

3. Zusammensetzung zur Verwendung nach Anspruch 1, wobei das SapC-DOPS über einen Behandlungszeitraum in eine Vielzahl von Dosen verabreicht wird.

4. Zusammensetzung zur Verwendung nach Anspruch 3, wobei der Behandlungszeitraum von etwa 20 bis etwa 40 aufeinanderfolgende Tage umfasst.

5. Zusammensetzung zur Verwendung nach Anspruch 3, wobei die Vielzahl von Dosen von SapC-DOPS über den Behandlungszeitraum von etwa 10 bis etwa 20 Dosen umfasst.

6. Zusammensetzung zur Verwendung nach Anspruch 3, wobei eine Dosis von SapC-DOPS von etwa 0,3 mg/kg bis etwa 1,2 mg/kg umfasst.

7. Zusammensetzung zur Verwendung nach Anspruch 1, wobei die fraktionierte Strahlentherapie eine Gesamtstrahlendosis von etwa 20 Gy bis etwa 80 Gy umfasst.

8. Zusammensetzung zur Verwendung nach Anspruch 1, wobei jede Fraktion eine Strahlungsdosis von etwa 1 Gy bis etwa 2 Gy umfasst.

9. Zusammensetzung zur Verwendung nach Anspruch 1, wobei der bösartige solide Tumor ein Gehirn-, Pankreas-, Lungen-, Blinddarm- oder Neuroblastomtumor ist.

10. Zusammensetzung zur Verwendung nach Anspruch 1, wobei das SapC-DOPS über einen intravenösen Weg verabreicht wird.

11. Zusammensetzung zur Verwendung nach Anspruch 1, wobei das SapC-DOPS in 10 bis 15 Dosen über einen Behandlungszeitraum von 28 Tagen verabreicht wird.

12. Zusammensetzung zur Verwendung nach Anspruch 1, wobei das Verfahren ferner die Verabreichung eines zusätzlichen Antikrebsmittels umfasst.

## Revendications

1. Composition comprenant de la saposine C et de la dioléoylphosphatidylsérine, SapC-DOPS, à utiliser dans un procédé de traitement d'une tumeur solide maligne chez un patient qui en a besoin, le procédé comprenant l'administration au patient d'une combinaison de SapC-DOPS et d'une radiothérapie fractionnée, dans laquelle la radiothérapie fractionnée comprend une pluralité de fractions de radiation, chacune desdites fractions délivrant une dose de radiation inférieure à une dose efficace pour inhiber la croissance de la tumeur lorsqu'elle est administrée seule, et dans laquelle une ou plusieurs fractions de la radiothérapie fractionnée sont administrées avant la SapC-DOPS, et :
(a) une ou plusieurs fractions supplémentaires sont administrées après la SapC-DOPS ;
(b) une ou plusieurs fractions supplémentaires sont administrées en même temps que la SapC-DOPS ; et/ou
(c) la SapC-DOPS et les fractions supplémentaires sont administrées en séquences superposées ou alternées.

2. Composition à utiliser selon la revendication 1, dans laquelle la radiothérapie fractionnée comprend de 20 à 40 fractions environ.

3. Composition à utiliser selon la revendication 1, dans laquelle la SapC-DOPS est administrée en plusieurs doses au cours d'une période de traitement.

4. Composition à utiliser selon la revendication 3, dans laquelle la période de traitement comprend d'environ 20 à environ 40 jours consécutifs.

5. Composition à utiliser selon la revendication 3, dans laquelle la pluralité de doses de SapC-DOPS comprennent d'environ 10 à environ 20 doses au cours de la période de traitement.

6. Composition à utiliser selon la revendication 3, dans laquelle une dose de SapC-DOPS est comprise entre environ 0,3 mg/kg et environ 1,2 mg/kg.

7. Composition à utiliser selon la revendication 1, dans laquelle la radiothérapie fractionnée comprend une dose totale de rayonnement d'environ 20 Gy à environ 80 Gy.

8. Composition à utiliser selon la revendication 1, dans laquelle chaque fraction comprend une dose de rayonnement d'environ 1 Gy à environ 2 Gy.

9. Composition à utiliser selon la revendication 1, dans laquelle la tumeur solide maligne est une tumeur cérébrale, pancréatique, pulmonaire, appendiculaire ou un neuroblastome.

10. Composition à utiliser selon la revendication 1, dans laquelle la SapC-DOPS est administrée par voie intraveineuse.

11. Composition à utiliser selon la revendication 1, dans laquelle la SapC-DOPS est administrée en 10 à 15 doses sur une période de traitement de vingt-huit jours.

12. Composition à utiliser selon la revendication 1, dans laquelle le procédé comprend en outre l'administration d'un agent anticancéreux supplémentaire.
